# EUROPEAN PATENT APPLICATION

(11) **EP 4 706 628 A1**
(43) Date of publication of application: **11.03.2026**
(21) Application number: 25195998.7
(22) Date of filing: 14.08.2025
(51) Int. Cl.: A61K 8/14, A61K 8/34, A61K 8/44, A61K 8/49, A61K 8/55, A61K 8/92, A61K 8/9728, A61K 8/9789, A61Q 5/02, A61Q 7/00

(54) **HAIR TONIC COMPOSITIONS AND METHODS FOR IMPROVING HAIR GROWTH OR PREVENTING HAIR LOSS**

(30) Priority: 14.08.2024 HK 32024095567; 29.10.2024 HK 32024098785
(71) Applicant: Mark Up Limited, Kowloon (HK)
(72) Inventor: CHAN, Helen Hei Ling, Tai Po, New Territories (HK)
(74) Representative: Cabinet Chaillot

(57) **Abstract**

In certain embodiments, herein are hair tonic compositions for improving hair growth and methods of use thereof. In certain embodiments, the present inventions are hair care tonic compositions for improving hair growth comprising nanosized ingredients. Other example embodiments are described herein. In certain embodiments, the present invention offers a highly effective and safe solution for addressing hair loss concerns.

## Description

### FIELD OF INVENTION

This application relates to hair tonic compositions and methods. In particular, this application pertains to hair tonic compositions and methods useful for preventing hair loss and improving hair growth.

### BACKGROUND OF INVENTION

Hair loss is a prevalent issue affecting a significant portion of the world population, often linked to emotional and psychological distress and other health concerns. While numerous products and treatments have been developed over decades to address this issue, existing solutions are generally perceived to have several limitations, which include concerns regarding their effectiveness and other undesirable side effects. Accordingly, there is a need for a new hair growth composition/treatment.

### SUMMARY OF INVENTION

Disclosed herein are novel hair tonic compositions and methods of use that are useful for hair growth, processes for preparing the compositions, methods of using the compositions, and intermediates used in preparing the compositions.

In some embodiments, the present inventions are hair tonic compositions, comprising all or part of active ingredients below: a plurality of nanosized particles, the nanosized particles comprises: *Phyllanthus emblica* Fruit Extract; *Zingiber officinale* (Ginger) Root Extract; *Sophora flavescens* Extract; *Eclipta prostrata* Extract; *Lithospermum erythrorhizon* Root Extract; *Bacopa monnieri* Extract; *Carthamus tinctorius* (Safflower) Flower Extract; *Polygoni multiflori radix praeparata* Extract; *Ligustrum lucidum* Extract; *Panax ginseng* Root Extract; *Mentha arvensis* Leaf Oil; Monosodium Glutamate; Cyanocobalamin; Caffeine; Rosemary Oil; and Menthol.

In some embodiments, the present inventions are hair tonic compositions, comprising nano vesicles from all or part of below: Unsaturated Phosphoslipids; Hydrogenated Phospholipids; Lecithins; Phosphatidylserine; Phosphatidylcholine; Phosphatidylglycerols; Phosphatidylethanolamines; Phosphatidic Acids; Pegylated Phospholipids; and Glycerophosphocholine.

In some embodiments, the present inventions are hair tonic compositions, comprising penetration enhancers from all or part of below: Glycerol; Ethyl Alcohol; Propyl Alcohol; Propylene Glycol; Butylene Glycol; Pentylene Glycol; Hexylene Glycol; Polysorbate 20; Polysorbate 60; Polysorbate 80; and Polyethylene Glycol 300.

In some embodiments, the present inventions are hair tonic compositions, comprising pH modulators from one or more of below: Ethylene Diamine; Arginine; Citric Acid; Ascorbic Acid; Sodium Ascorbate; and Sodium Hydroxide.

In some embodiments, the present inventions are methods of producing a hair tonic composition, comprising the steps of: a) nanosizing a first set of components with a high-pressure homogenizer to obtain a plurality of nanoparticles, wherein the components comprise the hair tonic composition of any one of the above embodiments; and b) adding the first set of components to a second set of components to produce the intermediate hair tonic composition or final hair tonic composition; wherein the nanosizing is performed with the high-pressure homogenizer at 100-1000 bar for 1-10 cycles.

In some embodiments, the present inventions are methods of uses of products that can be: 1) The hair tonic composition can be used alone; or 2) as combo use with the hair shampoo composition. The methods of uses of the hair tonic composition will be applying dosage on hair scalp, then massage for at least 1 minute. For combo use, apply dosage of the hair shampoo composition on hair scalp, then massage for at least 1 minute, followed by rinsing with water. After that, applying dosage of the hair tonic composition on the scalp. Massage for at least 1 minute. No need to rinse off.

There are many advantages of the invention. In some embodiments, the novel hair tonic compositions and methods of use support health and vitality of hair follies. In some embodiments, the novel hair tonic compositions and methods of use demonstrate remarkable improvements in hair growth and reduction in hair loss. In some embodiments, the novel hair tonic compositions and methods of use avoid harsh chemicals commonly found in conventional hair tonic products and thus reduce the risk of scalp irritation, dryness, and damage to the hair strands.

### BRIEF DESCRIPTION OF FIGURES

FIG. 1 is a flowchart diagram which illustrates the process for preparing a Nano Hair Tonic composition, according to an example embodiment.
FIGS. 2 and 3 are flowchart diagrams which illustrate the process for preparing the nanosized particles (Phases A-C) of a Nano Hair Shampoo composition and the subsequent process after nanosizing (further addition of Phases D-K) for preparing the Nano Hair Shampoo composition, respectively, according to an example embodiment.
FIG. 4 shows the experimental grouping of mice to investigate the hair growth efficacy of the Nano Hair Tonic composition and the Nano Hair Shampoo composition vs other commercial products.
FIG. 5A is a schematic diagram showing the standard operation procedures of a comparative study of mice receiving different treatments in Groups A (water), B (Nano Hair Tonic), D (Competitor Product I) and E (Competitor Product II), according to an example embodiment.
FIG. 5B is a schematic diagram showing the standard operation procedures of the comparative study of mice receiving treatment in Group C (Nano Hair Shampoo), according to the example embodiment of FIG. 5A.
FIG. 6 consists of a series of images of the back of mice receiving different treatments in Groups A-E, respectively, according to the example embodiment of FIGS. 5A-B.
FIG. 7 consists of a table and a bar chart diagram, showing the mean mouse hair lengths on Days 14, 21 and 28 in Groups A-E, according to the example embodiment of FIGS. 5A-B.
FIGS. 8A-E are a series of images showing hair follicles in the applied skin area of mice receiving different treatments in Groups A-E, respectively, on Days 7, 14, 21 and 28, according to the example embodiment of FIGS. 5A-B.
FIG. 9 consists of representative hair follicle histology images of the applied skin area of mice receiving different treatments in Groups A-C, respectively, on Day 28, according to the example embodiment of FIGS. 5A-B.

### DETAILED DESCRIPTION

As used herein and in the claims, the terms "comprising" (or any related forms such as "comprise" and "comprises"), "including" (or any related forms such as "include" or "includes"), "containing" (or any related forms such as "contain" or "contains"), means including the following elements but not excluding others. It shall be understood that for every embodiment in which the term "comprising" (or any related forms such as "comprise" and "comprises"), "including" (or any related forms such as "include" or "includes"), or "containing" (or any related forms such as "contain" or "contains") is used, this disclosure/application also includes alternate embodiments where the term "comprising", "including," or "containing," is replaced with "consisting essentially of" or "consisting of". These alternate embodiments that use "consisting of" or "consisting essentially of" are understood to be narrower embodiments of the "comprising", "including", or "containing" embodiments.

For example, alternate embodiments of "a composition comprising A, B, and C" would be "a composition consisting of A, B, and C" and "a composition consisting essentially of A, B, and C". Even if the latter two embodiments are not explicitly written out, this disclosure/application includes those embodiments. Furthermore, it shall be understood that the scopes of the three embodiments listed above are different.

For the sake of clarity, "comprising", "including", and "containing", and any related forms are open-ended terms which allow for additional elements or features beyond the named essential elements, whereas "consisting of" is a closed end term that is limited to the elements recited in the claims and excludes any element, step, or ingredient not specified in the claims.

For the sake of clarity, "characterized by" or "characterized in" (together with their related forms as described above) does not limit or change the nature of whether the list of terms following it is open or closed. For example, in a claim directed towards "a composition comprising A, B, C, and characterized in D, E, and F", the elements D, E, and F are still open-ended terms and the claim is meant to include other elements due to the use of the word "comprising" earlier in the claim.

"Consisting essentially of" limits the scope of a claim to the specified materials, components, or steps ("essential elements") that do not materially affect the essential characteristic(s) of the claimed invention. In some embodiments, the essential characteristics are the basic and novel characteristic(s) of the claimed invention. For example, in some embodiments, the essential elements of a composition of the disclosure can be "Xmg to Ymg" of compound A. Even if the composition includes additional excipients, as long as the additional excipients do not materially affect the essential characteristics of the compound, e.g., in compound A's ability to bind to XX target or to treat YY disease, then such embodiment that "consists essentially of compound A" still includes compositions with the aforementioned additional excipients.

As used herein, the singular forms "a", "an" and "the" are intended to include the plural forms as well, unless the context clearly indicates otherwise. Where a range is referred to in the specification, the range is understood to include each discrete point within the range. For example, 1-7 means 1, 2, 3, 4, 5, 6, and 7.

As used herein, the term "about" is understood as within a range of normal tolerance in the art and not more than ±10% of a stated value. By way of example only, about 50 means from 45 to 55 including all values in between. As used herein, the phrase "about" a specific value also includes the specific value, for example, about 50 includes 50.

As used herein and in the claims, an "effective amount" is an amount that is effective to achieve at least a measurable amount of a desired effect. For example, the amount may be effective to prevent hair loss and stimulate hair growth.

As used herein, the term "prevent", "preventing", "preventive", "preventative" or "prevention" refers to reducing the risk of the onset, relapse or spread of a condition.

As used herein, the term "homogenize(s)", "homogenizing" or "homogenization" refers to the process of mixing substances to achieve a substantially uniform distribution. As used herein, the term "homogenizer(s)" refers to instrument(s) or device(s) that carry out homogenization.

As used herein, the term "agitate(s)", "agitating" or "agitation" refers to an action of stirring, shaking, or vibrating substance(s). As used herein, the term "agitator(s)" refers to instrument(s) or device(s) for agitation.

As used herein, the term "nanosize", "nanosized" or "nanosizing" refers to reducing the size of substance(s) to achieve particle sizes in the range of 1 - 1.0 × 10⁵ nanometers (nm). In some examples, the nanosized particles are in the range of 10 - 1.0 × 10⁵ nanometers (nm). In some examples, the nanosized particles are in the range of 1-1000 nm.

As used herein, the term "nanoparticle(s)" refers to particle(s) with dimensions in the range of 1 - 1.0 × 10⁵ nm. In some examples, the nanoparticles are with dimensions in the range of 10 - 1.0 × 10⁵ nanometers (nm). In some examples, the nanoparticles are with dimensions in the range of 1-1000 nm.

As used herein, "extract(s)" refers to a concentrated form of substance(s).

The present inventions are hair tonic compositions that are not only highly effective in preventing hair loss and stimulating hair growth with positive user tolerance.

### NUMBERED EMBODIMENTS

Embodiment 1. A hair tonic composition, comprising all or part of active ingredients below: a plurality of nanosized particles, the nanosized particles comprises: *Phyllanthus emblica* Fruit Extract; *Zingiber officinale* (Ginger) Root Extract; *Sophora flavescens* Extract; *Eclipta prostrata* Extract; *Lithospermum erythrorhizon* Root Extract; *Bacopa monnieri* Extract; *Carthamus tinctorius* (Safflower) Flower Extract; *Polygoni multiflori radix praeparata* Extract; *Ligustrum lucidum* Extract; *Panax ginseng* Root Extract; *Mentha arvensis* Leaf Oil; Monosodium Glutamate; Cyanocobalamin; Caffeine; Rosemary Oil; and Menthol.

Embodiment 2. The hair tonic composition of embodiment 1, further comprising arginine.

Embodiment 3. The hair tonic composition of embodiments 1 or 2, wherein the active ingredients further comprise one or more ingredients selected from a group consistent of: Olive Oil; Grapeseed Oil; Ginger Oil; Chamomile Oil; Greentea Oil; Cedar Oil; Brown Seaweed Extract; *Dendrobii officinalis caulis* Extract; *Poria cocos* Extract; *Eucommia ulmoides* Bark Extract; Fucoidan; Fucoanxanthin; Amla; and Zinc Glycinate.

Embodiment 4. The hair tonic composition, comprising nano vesicles from all or part of below: Unsaturated Phosphoslipids; Hydrogenated Phospholipids; Lecithins; Phosphatidylserine; Phosphatidylcholine; Phosphatidylglycerols; Phosphatidylethanolamines; Phosphatidic Acids; Pegylated Phospholipids; and Glycerophosphocholine.

Embodiment 5. The hair tonic composition, comprising penetration enhancers from all or part of below: Glycerol; Ethyl Alcohol; Propyl Alcohol; Propylene Glycol; Butylene Glycol; Pentylene Glycol; Hexylene Glycol; Polysorbate 20; Polysorbate 60; Polysorbate 80; and Polyethylene Glycol 300.

Embodiment 6. The hair tonic composition, comprising pH modulators from one or more of below: Ethylene Diamine; Arginine; Citric Acid; Ascorbic Acid; Sodium Ascorbate; and Sodium Hydroxide.

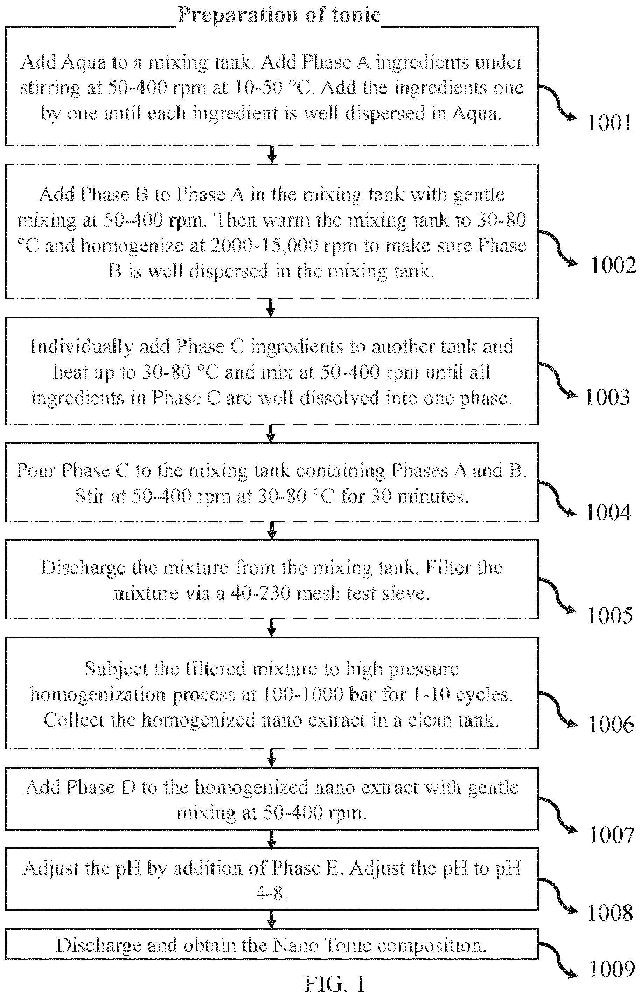

Embodiment 7. The hair tonic composition of any one of embodiments 1-6, wherein the hair tonic composition is an intermediate hair tonic composition or final hair tonic composition. In some embodiments, the intermediate hair tonic composition is/are the product(s) of step 1001, 1002, 1003, 1004, 1005, 1006, 1007, or 1008 in Figure 1. In some embodiments, the final hair tonic composition is the product of step 1009 in Figure 1.

Embodiment 8. The hair tonic composition of any one of embodiments 1-7, wherein the plurality of nanoparticles is 10 - 1.0 × 10⁵ nm in diameter.

Embodiment 9. A method of producing a hair tonic composition, comprising the steps of: a) nanosizing a first set of components with a high-pressure homogenizer to obtain a plurality of nanoparticles, wherein the components comprise the hair tonic composition of any one of embodiments 1, 3-5; and b) adding the first set of components to a second set of components to produce the intermediate hair tonic composition or final hair tonic composition; wherein the nanosizing is performed with the high-pressure homogenizer at under 100-1000 bar for 1-10 cycles. In some embodiments, the first set of components comprises all or part of the ingredients of Phase A, Phase B, and Phase C listed in Table 1A. In some embodiments, the second set of components comprises all or part of the ingredients of Phase D, and Phase E listed in Table 1A.

Embodiment 10. The method of embodiment 9, wherein the hair tonic composition further comprises arginine.

Embodiment 11. The method of embodiments 9 or 10, wherein the hair tonic composition further comprises the hair tonic composition of embodiment 6.

Embodiment 12. The method of any one of embodiments 9-11, wherein the first set of components is filtered via a 40-230 mesh test sieve before nanosizing.

Embodiment 13. The method of producing the hair tonic composition of any one of embodiments 9-12, prior to step a), the method comprises the steps of: i) adding aqua to a first mixing tank and then adding individually a first subset of the first set of components under stirring at 50-400 rpm at 10-50 °C, producing a first mixture; ii) adding a second subset of the first set of components to the first mixing tank with gentle mixing at 50-400 rpm, and then warming the first mixing tank to 30-80 °C and homogenizing at 2000-15,000 rpm, producing a second mixture; iii) adding individually a third subset of the first set of components to a second mixing tank and heating up to 30-80 °C and mixing at 50-400 rpm, producing a third mixture; iv) pouring the third mixture to the second mixture and then stirring at 50-400 rpm at 30-80 °C for 30 minutes, producing a fourth mixture; v) filtering the fourth mixture via a 40-230 mesh test sieve; and wherein the nanosizing is performed at 100-1000 bar for 1-10 cycles. In some embodiments, the first subset of the first set of components comprises all or part of the ingredients of Phase A listed in Table 1A. In some embodiments, the second subset of the first set of components comprises all or part of the ingredients of Phase B listed in Table 1A. In some embodiments, the third subset of the first set of components comprises all or part of the ingredients of Phase C listed in Table 1A.

Embodiment 14. The method of producing the hair tonic composition of any one of embodiments 9-13, wherein in step b), the method comprises the steps of: vi) adding a first subset of the second set of components to the fourth mixture homogenized with the high-pressure homogenizer with gentle mixing at 50-400 rpm, producing a fifth mixture; and vii) adding a second subset of the second set of components to the fifth mixture to adjust the pH to 4-8, and discharging to produce the hair tonic composition. In some embodiments, the first subset of the second set of components comprises all or part of the ingredients of Phase D listed in Table 1A. In some embodiments, the second subset of the second set of components comprises all or part of the ingredients of Phase E listed in Table 1A.

Embodiment 15. The method of any one of embodiments 9-14, wherein the plurality of nanoparticles is 10 - 1.0 × 10⁵ nm in diameter.

Embodiment 16. The method of embodiment 15, wherein the plurality of nanoparticles is 122-1000 nm in diameter.

Embodiment 17. A hair tonic composition for improving hair growth or preventing hair loss, wherein the intermediate hair tonic composition or final hair tonic composition comprises: 0.01-1.00% Monosodium Glutamate; 0.01-1.00% Chitosan; 1.00-10.00% *Phyllanthus emblica* Fruit Extract; 1.00-10.00% *Zingiber officinale* Root Extract; 1.00-10.00% *Sophora flavescens* Extract; 1.00-10.00% *Eclipta prostrata* Extract; 0.01-10.00% *Lithospermum erythrorhizon* Root Extract; 0.01-10.00% Cyanocobalamin; 0.01-10.00% Caffeine; 0.01-10.00% *Bacopa monnieri* Extract; 0.01-10.00% *Carthamus tinctorius* Flower Extract; 0.01-10.00% *Polygoni multiflori radix praeparata* Extract; 0.01-10.00% *Ligustrum lucidum* Extract; 0.01-10.00% *Panax ginseng* Root Extract; 0.05-10.00% Lecithin; 0.01-10.00% *Mentha arvensis* Leaf Oil; 0.01-10.00% PEG-40 Hydrogenated Castor Oil; 1.00-5.00% Polysorbate 20; 0.01-10.00% Rosemary Oil; 0.01-10.00% Menthol; 1.00-10.00% Glycerin; 1.00-10.00% Butylene Glycol; 0.10-10.00% Ethylhexylglycerin; Capryloyl Glycine; 1,2-Hexanediol; and Butylene Glycol; 0.10-10.00% Pentylene Glycol; and 0.01-10.00% Arginine; and water adding up to 100%.

Embodiment 18. The hair tonic composition of embodiment 17, wherein the hair tonic composition is produced by a process comprising the steps of: a) nanosizing a first set of components with a high-pressure homogenizer to obtain a plurality of nanoparticles, wherein the components comprise: *Phyllanthus emblica* Fruit Extract; *Zingiber officinale* (Ginger) Root Extract; *Sophora flavescens* Extract; *Eclipta prostrata* Extract; *Lithospermum erythrorhizon* Root Extract; *Bacopa monnieri* Extract; *Carthamus tinctorius* (Safflower) Flower Extract; *Polygoni multiflori radix praeparata* Extract; *Ligustrum lucidum* Extract; *Panax ginseng* Root Extract; *Mentha arvensis* Leaf Oil; Monosodium Glutamate; Cyanocobalamin; Caffeine; Rosemary Oil; Menthol; and b) adding the first set of components to a second set of components to produce the intermediate hair tonic composition or final hair tonic composition; wherein the nanosizing is performed with the high-pressure homogenizer at under 100-1000 bar for 1-10 cycles.

Embodiment 19. Use of an effective amount of the hair tonic composition of any one of embodiments 1-8, 17-18 or the hair tonic composition produced by the method of any one of embodiments 9-16 on hair scalp, wherein after the hair tonic composition is applied on the hair scalp, the hair scalp is massaged for at least 1 minute.

Embodiment 20. The use of embodiment 19, further comprising an effective amount of a hair shampoo composition on the hair scalp.

Embodiment 21. Use of an effective amount of a combination of the hair tonic composition of any one of embodiments 1-8, 17-18 or the hair tonic composition produced by the method of any one of embodiments 9-16 and a hair shampoo composition on hair scalp, wherein after the hair shampoo composition is applied on the hair scalp, the hair scalp is massaged for at least 1 minute, followed by rinsing the hair scalp with water; then the hair tonic composition is applied on the hair scalp, the hair scalp is massaged for at least 1 minute.

### EXAMPLES

Provided herein are examples that describe in more detail certain embodiments of the present disclosure. The examples provided herein are merely for illustrative purposes and are not meant to limit the scope of the invention in any way. All references given below and elsewhere in the present application are hereby included by reference.

### Example 1: Example method of producing a Nano Tonic composition

Table 1A provides a list of ingredients in a Nano Tonic composition of the present disclosure. The Nano Tonic composition in Example 1 is prepared from the ingredients listed in Table 1A.

**Table 1A. List of ingredients in a Nano Tonic composition.**

| Item | Phase | Ingredients | Range % w/w of tonic composition |
|---|---|---|---|
| 1 | A | Aqua/Water | To 100.00 |
| 2 | A | Monosodium Glutamate | 0.01-1.00 |
| 3 | A | Chitosan | 0.01-1.00 |
| 4 | A | *Phyllanthus emblica* Fruit Extract | 1.00-10.00 |
| 5 | A | *Zingiber officinale* (Ginger) Root Extract | 1.00-10.00 |
| 6 | A | *Sophora flavescens* Extract | 1.00-10.00 |
| 7 | A | *Eclipta prostrata* Extract | 1.00-10.00 |
| 8 | A | *Lithospermum erythrorhizon* Root Extract | 0.01-10.00 |
| 9 | A | Cyanocobalamin | 0.01-10.00 |
| 10 | A | Caffeine | 0.01-10.00 |
| 11 | A | *Bacopa monnieri* Extract | 0.01-10.00 |
| 12 | A | *Carthamus tinctorius* (Safflower) Flower Extract | 0.01-10.00 |
| 13 | A | *Polygoni multiflori radix praeparata* Extract | 0.01-10.00 |
| 14 | A | *Ligustrum lucidum* Extract | 0.01-10.00 |
| 15 | A | *Panax ginseng* Root Extract | 0.01-10.00 |
| 16 | B | Lecithin | 0.05-10.00 |
| 17 | C | *Mentha arvensis* Leaf Oil | 0.01-10.00 |
| 18 | C | PEG-40 Hydrogenated Castor Oil | 0.01-10.00 |
| 19 | C | Polysorbate 20 | 1.00-5.00 |
| 20 | C | Rosemary Oil | 0.01-10.00 |
| 21 | C | Menthol | 0.01-10.00 |
| 22 | C | Glycerin | 1.00-10.00 |
| 23 | C | Butylene Glycol | 1.00-10.00 |
| 24 | D | Ethylhexylglycerin | 0.10-10.00 |
| | | Capryloyl Glycine | |
| | | 1,2-Hexanediol | |
| | | Butylene Glycol | |
| 25 | D | Pentylene Glycol | 0.10-10.00 |
| 26 | E | Arginine | 0.01-10.00 |

The 100.00% of the final volume of tonic composition refers to the total volume when all ingredients are included and water is added to make it 100%.

The ingredients from Table 1A are incorporated sequentially in different phases, denoted as Phases A-E in Table 1A, to form the Nano Tonic composition. Example 1 outlines the process of preparing the Nano Tonic composition.

Now referring to FIG. 1. In this example, the Nano Tonic composition is prepared by first adding aqua to a mixing tank and then adding ingredients in Phase A under stirring at 50-400 rpm at 10-50 °C. The Phase A ingredients are added one by one until each ingredient is well dispersed in the aqua (Step 1001). Ingredients from Phase B are then added into Phase A in the mixing tank with gentle mixing at 50-400 rpm, and the ingredients are warmed to 30-80 °C and homogenized at 2000-15,000 rpm until all the materials are well dispersed (Step 1002). In another tank, Phase C ingredients are added individually and heated up to 30-80 °C and mixed at 50-400 rpm until all ingredients in Phase C are well dissolved into one phase (Step 1003). The Phase C pre-mixture is then gently incorporated into the mixing tank containing Phases A and B while continuing to stir at 50-400 rpm at 30-80 °C for 30 minutes (Step 1004). The mixture containing Phases A, B and C is discharged from the mixing tank and filtered via a 40-230 mesh test sieve (Step 1005), and is then subjected to high pressure homogenization process at 100-1000 bar for 1-10 cycles to obtain nanosized particles. After nanosizing, the homogenized nanosized particles are collected in a clean tank (Step 1006). The ingredients of Phase D are added to the homogenized nanosized particles, accompanied by gentle mixing at 50-400 rpm (Step 1007). In some embodiments, 0.10-10.00% of the tonic composition comprises Phase D ingredients selected from the group consisting of Ethylhexylglycerin, Capryloyl Glycine, 1,2-Hexanediol and Butylene Glycol. Ingredient of Phase E is added to the mixture to adjust the pH to 4-8 (Step 1008). Lastly, the final mixture encompassing Phases A through E is discharged. A Nano Tonic composition is obtained (Step 1009).

Tables 1B and 1C provide lists of active ingredients and components of nano vesicles in a Nano Tonic composition in some embodiments. The active ingredients and nano vesicles are useful for improving hair growth and/or preventing hair loss, supporting health and vitality of hair follicles, and/or reducing the risk of scalp irritation, dryness, and damage to the hair strands.

**Table 1B. List of active ingredients.**

| Names of Active Incredients | Usage Ranges |
|---|---|
| *Phyllanthus emblica* Fruit Extract | 1-10% |
| *Zingiber officinale* (Ginger) Root Extract | 1-10% |
| *Sophora flavescens* Extract | 1-10% |
| *Eclipta protrata* Extract | 1-10% |
| *Lithospermum erythrorhizon* Root Extract | 0.01-10% |
| Cyanocobalamin | 0.01-10% |
| Caffeine | 0.01-10% |
| *Bacopa monnieri* Extract | 0.01-10% |
| Monosodium Glutamate | 0.01-1% |
| *Carthamus tinctorius* Flower Extract | 0.01-1% |
| *Polygoni multiflori radix praeparata* Extract | 0.01-1% |
| *Ligustrum lucidum* Extract | 0.01-1% |
| *Panax ginseng* Root Extract | 0.01-1% |
| *Mentha arvensis* Leaf Oil | 0.01-1% |
| Rosemary Oil | 0.01-1% |
| Olive Oil | 0.01-1% |
| Grapeseed Oil | 0.01-1% |
| Ginger Oil | 0.01-1% |
| Chamomile Oil | 0.01-1% |
| Greentea Oil | 0.01-1% |
| Cedar Oil | 0.01-1% |
| Brown Seaweed Extract | 0.01-1% |
| *Dendrobii officinalis caulis* Extract | 0.01-1% |
| *Poria cocos* Extract | 0.01-1% |
| *Eucommia ulmoides* Bark Extract | 0.01-1% |
| Fucoidan | 0.01-10% |
| Fucoanxanthin | 0.01-10% |
| Amla | 0.01-10% |
| Menthol | 0.01-10% |
| Zinc Glycinate | 0.01-1% |

In addition to the active ingredients cited in Table 1A, one or more ingredients can be added/replaced by other ingredients of Table 1B to achieve similar effect shown in this application.

**Table 1C. List of components of nano vesicles.**

| Names of Components of Nano Vesicles | Usage Ranges |
|---|---|
| Unsaturated Phosphoslipids | 0.05-10% |
| Hydrogenated Phospholipids | 0.05-10% |
| Lecithins | 0.05-10% |
| Phosphatidylserine | 0.05-10% |
| Phosphatidylcholine | 0.05-10% |
| Phosphatidylglycerols | 0.05-10% |
| Phosphatidylethanolamines | 0.05-10% |
| Phosphatidic Acids | 0.05-10% |
| Pegylated Phospholipids | 0.05-10% |
| Glycerophosphocholine | 0.05-10% |

In addition to the nano vesicles cited in Table 1A, one or more nano vesicles can be added/replaced by other nano vesicles of Table 1C to achieve similar effect shown in this application.

In some embodiments, penetration enhancers used in a Nano Tonic composition are selected from a group comprising Glycerol, Ethyl Alcohol, Propyl Alcohol, Propylene Glycol, Butylene Glycol, Pentylene Glycol, Hexylene Glycol, Polysorbate 20, Polysorbate 60, Polysorbate 80, and Polyethylene Glycol 300. Without further limitation, it is understandable other penetration enhancers can also be used for the same functions.

In some embodiments, pH modulators used in a Nano Tonic composition are selected from a group comprising Ethylene Diamine, Arginine, Citric Acid, Ascorbic Acid, Sodium Ascorbate, and Sodium Hydroxide. Without further limitation, it is understandable other pH modulators can also be used for the same functions.

### Example 2: Example method of producing a Nano Shampoo composition

Table 2A provides a list of ingredients in a Nano Shampoo composition of the present disclosure. The Nano Shampoo composition in Example 2 is prepared from the ingredients listed in Table 2A.

**Table 2A. List of ingredients in a Nano Shampoo composition.**

| Item | Phase | Ingredients | Range % w/w of shampoo composition |
|---|---|---|---|
| 1 | A | Lecithin | 0.05-10.00 |
| 2 | A | Glycerol | 1.00-10.00 |
| 3 | A | Butylene Glycol | 1.00-10.00 |
| 4 | A | Menthol | 0.01-10.00 |
| 5 | A | Rosemary Oil | 0.01-10.00 |
| 6 | B | Aqua/Water | 10.00-50.00 |
| 7 | B | Monosodium Glutamate | 0.01-1.00 |
| 8 | B | *Polygoni multiflori radix praeparata* Extract | 0.01-10.00 |
| | | Aqua/Water | |
| | | *Ligustrum lucidum* Extract | |
| | | *Eclipta prostrata* Extract | |
| | | *Ganoderma lucidum* (Mushroom) Extract | |
| | | *Angelica archangelica* Extract | |
| | | *Achyranthes bidentata* Extract | |
| | | *Poria cocos* Extract | |
| | | *Eucommia ulmoides* Bark Extract | |
| 9 | B | *Panax ginseng* Root Extract | 0.01-10.00 |
| 10 | B | Caffeine | 0.01-10.00 |
| 11 | C | Phenoxyethanol | 0.10-1.00 |
| | | Ethylhexylglycerin | |
| 12 | D | Aqua/Water | To 100.00 |
| 13 | D | Polyquaternium-10 | 0.01-10.00 |
| | | Sodium Chloride | |
| 14 | D | Guar Hydroxypropyltrimonium Chloride | 0.01-10.00 |
| 15 | D | Citric Acid | 0.01-10.00 |
| 16 | E | Ammonium Lauryl Sulfate | 1.00-50.00 |
| 17 | F | Cocamide MEA | 1.00-10.00 |
| 18 | F | Decyl Glucoside | 1.00-10.00 |
| 19 | G | Glycol Distearate | 1.00-10.00 |
| | | Laureth-4 | |
| | | Cocamidopropyl Betaine | |
| | | Formic Acid | |
| 20 | G | Panthenol | 0.10-1.00 |
| 21 | G | Tetrasodium EDTA | 0.01-1.00 |
| 22 | G | Hydrolyzed Wheat Protein | 0.10-10.00 |
| | | Sodium Benzoate | |
| 23 | G | Dicaprylyl Ether | 0.10-1.00 |
| 24 | G | Steartrimonium Chloride | 1.00-10.00 |
| 25 | G | Polyquaternium-6 | 1.00-10.00 |
| 26 | H | Phenoxyethanol | 0.10-1.00 |
| | | Ethylhexylglycerin | |
| 27 | H | Sorbitan Caprylate | 1.00-10.00 |
| 28 | J | Acrylates Copolymer | 2.00-20.00 |
| 29 | K | Arginine | 1.01-20.00 |

The 100.00% of the final volume of shampoo composition refers to the total volume when all ingredients are included and water is added to make it 100%.

The ingredients from Table 2A are incorporated sequentially in different phases, denoted as Phases A-K in Table 2A, to form the Nano Shampoo composition. Example 2 outlines the process of preparing the Nano Shampoo composition.

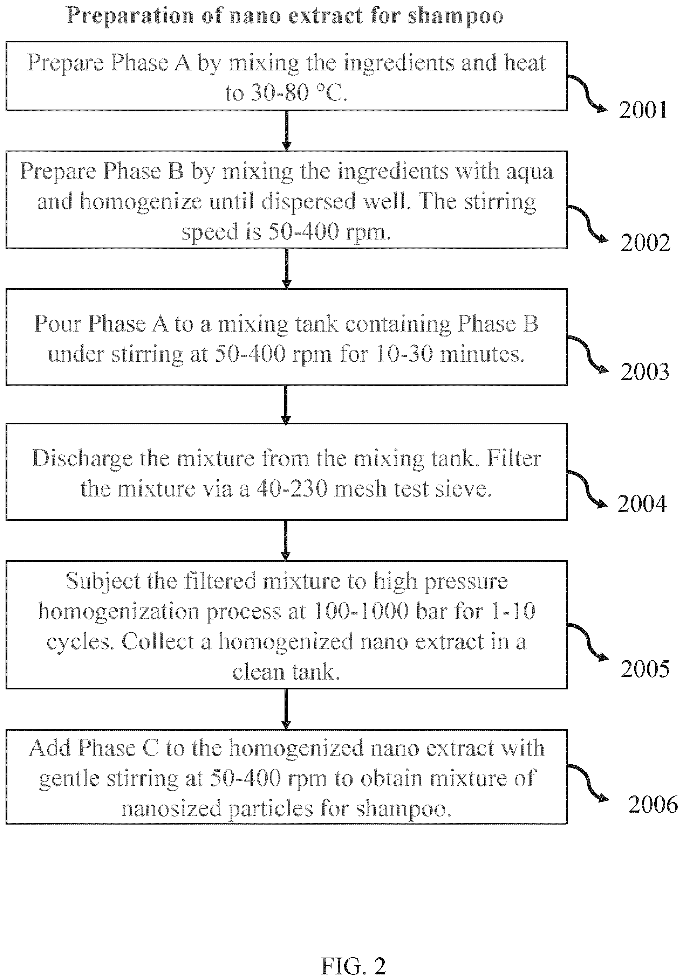

Now referring to FIG. 2. In this example, the preparation of nanosized particles for the Nano Shampoo composition starts by mixing the ingredients in Phase A in a tank and heating the mixture to 30-80 °C (Step 2001). In another tank, the Phase B ingredients are mixed with aqua and homogenized until dispersed well with gentle stirring at 50-400 rpm (Step 2002). In some embodiments, 0.01-10.00% of the shampoo composition comprises Phase B ingredients selected from the group consisting of *Ligustrum lucidum* Extract, *Eclipta prostrata* Extract, *Ganoderma lucidum* (Mushroom) Extract, *Angelica archangelica* Extract, *Achyranthes bidentata* Extract, *Poria cocos* Extract and *Eucommia ulmoides* Bark Extract. Phase A is then poured to Phase B under stirring at 50-400 rpm for 10-30 minutes (Step 2003). The mixture containing Phases A and B is discharged from the tank and filtered via a 40-230 mesh test sieve (Step 2004), and is then subjected to high pressure homogenization process at 100-1000 bar for 1-10 cycles. After nanosizing, a homogenized nano extract is collected in a clean tank (Step 2005). Phase C ingredients are added to the homogenized nano extract with gentle stirring at 50-400 rpm to obtain the mixture of nanosized particles for the Nano Shampoo composition (Step 2006). In some embodiments, 0.10-1.00% of the shampoo composition comprises Phase C ingredients selected from the group consisting of Phenoxyethanol and Ethylhexylglycerin.

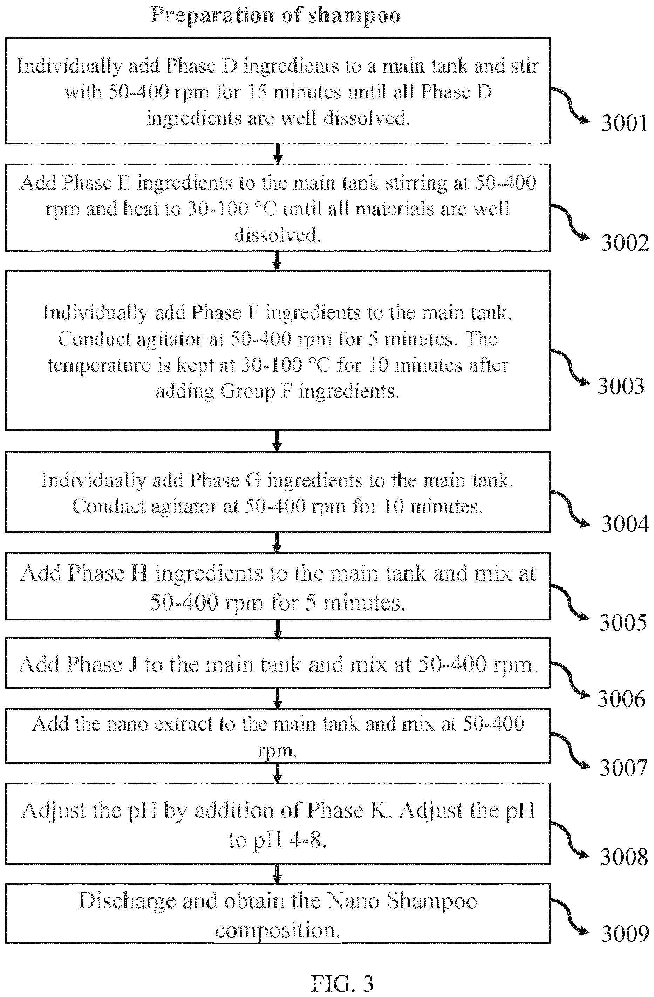

Now referring to FIG. 3. In a main tank, the ingredients in Phase D are individually added and stirred with 50-400 rpm for 15 minutes until all Phase D ingredients are well dissolved (Step 3001). In some embodiments, 0.01-10.00% of the shampoo composition comprises Phase D ingredients selected from the group consisting of Polyquaternium-10 and Sodium Chloride. Phase E ingredients are added to the main tank and the mixture is stirred at 50-400 rpm and heated to 30-100 °C until all materials are well dissolved (Step 3002). Phase F ingredients are added individually to the main tank containing Phases D and E under agitation at 50-400 rpm for 5 minutes using an agitator. The mixture is then kept at 30-100 °C for 10 minutes (Step 3003). Phase G ingredients are then added individually to the main tank under agitation at 50-400 rpm for 10 minutes using an agitator (Step 3004). In some embodiments, 1.00-10.00% of the shampoo composition comprises Phase G ingredients selected from the group consisting of Glycol Distearate, Laureth-4, Cocamidopropyl Betaine and Formic Acid. In some embodiments, 0.10-10.00% of the shampoo composition comprises Phase G ingredients selected from the group consisting of Hydrolyzed Wheat Protein and Sodium Benzoate. Phase H ingredients are incorporated to the main tank and mixed at 50-400 rpm for 5 minutes (Step 3005), followed by the addition of Phase J ingredients and then mixing at 50-400 rpm (Step 3006). In some embodiments, 0.10-1.00% of the shampoo composition comprises Phase G ingredients selected from the group consisting of Phenoxyethanol and Ethylhexylglycerin. The nanosized particles from Phases A, B and C obtained by an example method illustrated in FIG. 2 are introduced to the main tank which now contains ingredients from Phases D-J. The mixture is mixed at 50-400 rpm (Step 3007). Subsequently, ingredients of Phase K are added to the mixture to adjust the pH to 4-8 (Step 3008). Lastly, the final mixture encompassing Phases A through K is discharged. A Nano Shampoo composition is obtained (Step 3009).

Tables 2B and 2C provide lists of active ingredients and components of nano vesicles in a Nano Shampoo composition in some embodiments. The active ingredients and nano vesicles are useful for improving hair growth and/or preventing hair loss, supporting health and vitality of hair follicles, and/or reducing the risk of scalp irritation, dryness, and damage to the hair strands.

**Table 2B. List of active ingredients.**

| Name of Active Ingredients | Usage Ranges |
|---|---|
| *Polygoni multiflori radix praeparata* Extract | 0.01-10% |
| *Ligustrum lucidum* Extract | 0.01-10% |
| *Eclipta prostrata* Extract | 0.01-10% |
| *Ganoderma lucidum* (Mushroom) Extract | 0.01-10% |
| *Zingiber officinale* (Ginger) Root Extract | 0.01-10% |
| Brown seaweed Extract | 0.01-10% |
| *Angelica archangelica* Extract | 0.01-10% |
| *Achyranthes bidentata* Extract | 0.01-10% |
| *Dendrobii officinalis caulis* Extract | 0.01-10% |
| *Poria cocos* Extract | 0.01-10% |
| *Eucommia ulmoides* Bark Extract | 0.01-10% |
| *Panax ginseng* Root Extract | 0.01-10% |
| Fucoidan | 0.01-10% |
| Fucoanxanthin | 0.01-10% |
| Amla | 0.01-10% |
| Menthol | 0.01-10% |
| Olive Oil | 0.01-10% |
| Grapeseed Oil | 0.01-10% |
| Ginger Oil | 0.01-10% |
| Rosemary Oil | 0.01-10% |
| Chamomile Oil | 0.01-10% |
| Greentea Oil | 0.01-10% |
| Cedar Oil | 0.01-10% |
| Monosodium Glutamate | 0.01-1% |
| Zinc Glycinate | 0.01-1% |
| Caffeine | 0.01-10% |

In addition to the active ingredients cited in Table 2A, one or more ingredients can be added/replaced by other ingredients of Table 2B to achieve similar effect shown in this application.

**Table 2C. List of components of nano vesicles**

| Names of Components of Nano Vesicles | Usage Ranges |
|---|---|
| Unsaturated Phosphoslipids | 0.05-10% |
| Hydrogenated Phospholipids | 0.05-10% |
| Lecithins | 0.05-10% |
| Phosphatidylserine | 0.05-10% |
| Phosphatidylcholine | 0.05-10% |
| Phosphatidylglycerols | 0.05-10% |
| Phosphatidylethanolamines | 0.05-10% |
| Phosphatidic Acids | 0.05-10% |
| Pegylated Phospholipids | 0.05-10% |
| Glycerophosphocholine | 0.05-10% |

In addition to the nano vesicles cited in Table 2A, one or more nano vesicles can be added/replaced by other nano vesicles of Table 2C to achieve similar effect shown in this application.

In some embodiments, penetration enhancers used in a Nano Shampoo composition are selected from a group comprising Glycerol, Ethyl Alcohol, Propyl Alcohol, Propylene Glycol, Butylene Glycol, Pentylene Glycol, Hexylene Glycol, Polysorbate 20, Polysorbate 80, and 1-Tetradecane. Without further limitation, it is understandable other penetration enhancers can also be used for the same functions.

In some embodiments, pH modulators used in a Nano Shampoo composition are selected from a group comprising Ethylene Diamine, Arginine, Citric Acid, and Sodium Hydroxide. Without further limitation, it is understandable other pH modulators can also be used for the same functions.

### Example 3: Mean particle sizes of nanosized particles of the Nano Tonic composition and the Nano Shampoo composition

The mean particle sizes of the nanosized particles of the Nano Tonic composition and the Nano Shampoo composition under various pressures used for high pressure homogenization (HPH) and number of HPH cycles were measured by dynamic light scattering method and are shown in Tables 3 and 4, respectively.

**Table 3. Mean particle sizes of the nanosized particles of the Nano Tonic composition**

| Pressure used for high pressure homogenization | No. of cycles | Mean particle size (nm) |
|---|---|---|
| 100 | 1 | 177.1 |
| 500 | 1 | 131.5 |
| 500 | 2 | 160.9 |
| 500 | 5 | 205.7 |
| 1000 | 5 | 174.5 |
| 100 | 5 | 151.9 |
| 100 | 10 | 149.7 |
| 100 | 15 | 145.3 |
| 200 | 5 | 141.3 |
| 200 | 10 | 146.2 |
| 200 | 15 | 145.1 |
| 300 | 5 | 149.5 |
| 300 | 10 | 145.5 |
| 300 | 15 | 154.4 |

**Table 4. Mean particle sizes of the nanosized particles of the Nano Shampoo composition**

| Pressure used for high pressure homogenization | No. of cycles | Mean particle size (nm) |
|---|---|---|
| 500 | 1 | 626.1 |
| 500 | 2 | 357.2 |
| 500 | 3 | 373 |
| 500 | 3 | 415.2 |
| 500 | 4 | 394.0 |
| 500 | 5 | 397.6 |

### Example 4: Comparative Studies using the Nano Tonic Composition and the Nano Shampoo Composition

Example 4 shows a comparative study to investigate and compare the efficacies of the Nano Tonic composition (prepared based on the method of Example 1) and Nano Shampoo composition (prepared based on the method of Example 2) in promoting hair growth, to those of two commercially available benchmark products.

### 4.1 Materials

- Nano Tonic Composition prepared based on the method of Example 1
- Nano Shampoo composition prepared based on the method of Example 2
- Competitor product I
- Competitor product II
- Sucrose-PBS solution (30%)
- 4% Paraformaldehyde Fix Solution was purchased from Nanjing Fandun Biotechnology Center (Nanjing, China)
- Tissue-Tek OCT compound was purchased from Sakura Finetek USA, Inc. (Torrance, CA, USA)

### Ingredients of Competitor product I:

- Water
- Alcohol
- Pentylene Glycol
- *Panax ginseng* Root Extract
- PEG-40 Hydrogenated Castor Oil
- Hydroxylethylcellulose
- Acetyl Tyrosine
- *Arctium Majus* Root Extract
- Hydrolyzed Soy Protein
- Polyquaternium-11
- PEG-12 Dimethicone
- Calcium Pantothenate
- Zinc Gluconate
- Fragrance
- Ethylhexylglycerin
- Niacinamide
- Ornithine HCl
- *Malus domestica* Fruit Cell Culture Extract
- Butylene Glycol
- Citrulline
- Dipotassium Glycyrrhizate
- Glucosamine HCl
- Phenoxylethanol
- Xanthan Gum
- Glycerin
- Biotin
- PEG/PPG/Polybutylene Glycol-8/5/3 Glycerin
- Lecithin
- Majorana Leaf Extract
- Isomalt
- *Humulus lupulus* (Hops) Flower Extract
- *Swertia japonica* Extract
- Algae Extract
- *Eriobotrya japonica* Leaf Extract
- Glycerylamidoethyl Methacrylate/Steryl Methscrylate Copolymer
- *Phellodendron amurense* Bark Extract
- Soluble Proteoglycan
- *Argania spinosa* Callus Culture Extract
- Sodium Benzoate

### Ingredients of Competitor Product 2:

- Water
- Alcohol
- Butylene Glycol
- PEG-40 Hydrogenated Castor Oil
- Menthol
- Xanthan Gum
- Caffeine
- Hydroxypropyl Methylcellulose Stearoxy Ether
- Fragrance
- Disodium EDTA
- Dipotassium Glycyrrhizate
- *Panax ginseng* Root Extract
- *Swertia japonica* Extract
- *Eriobotrya japonica* Leaf Extract
- *Chamomilla recutita* (Matricaria) Flower Extract
- *Symphytum officinale* Leaf Extract
- *Zingiber officinale* (Ginger) Root Extract
- *Malva sylvestris* (Mallow) Flower Extract
- *Simmondsia chinensis* (Jojoba) Seed Oil
- Sea Salt
- *Olea europaea* (Olive) Fruit Oil
- Hydrolyzed Soy Protein
- *Butyrospermum parkii* (Shea Butter)
- *Argania spinosa* Kernel Oil
- Soluble Collagen
- Hydrolyzed Collagen
- Quaternium-33
- *Poria cocos* Extract
- Retinyl Palmitate
- *Helianthus annuus* (Sunflower) Seed Oil
- Betaine
- Sodium PCA
- Glycerin
- Polyglyceryl-10 Myristate
- Phenoxyethanol
- Lanolin Acid
- Sorbitol
- Ceramide 2
- Cholesterol
- *Hypnea musciformis* Extract
- *Undaria pinnatifida* Extract
- Serine
- Hydrolyzed Silk
- Tocopherol
- Glycine
- BHT
- Glutamic Acid
- *Hizikia fusiforme* Extract
- Alanine
- Yeast Extract
- Lysine
- Arginine
- *Pueraria lobata* Root Extract
- Threonine
- *Rosmarinus officinalis* (Rosemary) Leaf Extract
- Proline
- *Pinus sylvestris* Cone Extract
- Methylparaben
- *Humulus lupulus* (Hops) Flower Extract
- *Glycine soja* (Soybean) Seed Extract
- *Equisetum arvense* Extract
- *Citrus limon* (Lemon) Fruit Extract
- *Chlorella vulgaris* Extract
- *Aloe barbadensis* Leaf Extract
- *Cichorium intybus* (Chicory) Leaf Extract
- *Apium graveolens* (Celery) Extract
- *Brassica oleracea* Italica (Broccoli) Extract
- *Citrus junos* Fruit Extract
- Propylparaben

### 4.2 Devices

Photos of mouse backs were taken using an iPhone 13 which was fixed on a platform. Hair follicles were observed with an AoBaoKang^{®} HD digital detecting instrument (W-1002) equipped with a TNx200 lens. Lengths of mouse hairs were measured using an ERD-016 Digital Caliper. A RWD Gas Evacuation Apparatus was used to anesthetize the mice with isoflurane. Frozen sections were performed on a Leica CM3050S Cryotome with CryoJane Tape Transfer System. Skin tissue sessions were observed on an ECLIPSE Ti-E inverted microscope combined with NIS-Elements imaging software.

### 4.3 Experimental Mice and Grouping

Eight-week-old male C57BL/6 mice were purchased from the Laboratory Animal Services Center of the Chinese University of Hong Kong and housed in a required room under controlled conditions (23-27 °C; 45-55% humidity; 12-12 h light-dark cycle). Experimental procedures were performed with the prior approval of Animal Ethics Committee (Ref. No.: 22-306-MIS) at The Chinese University of Hong Kong (Hong Kong SAR, China).

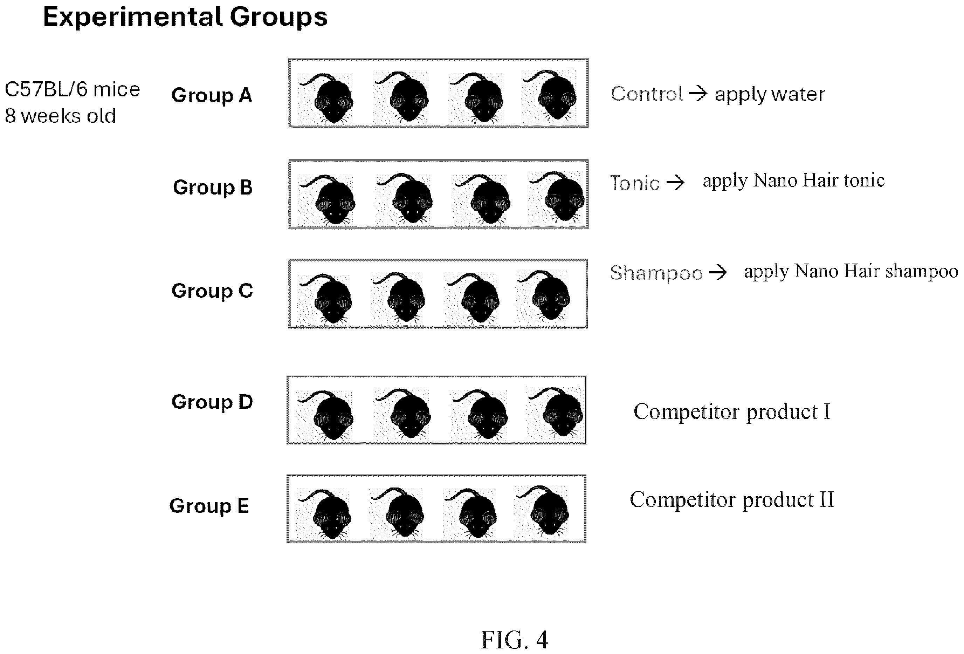

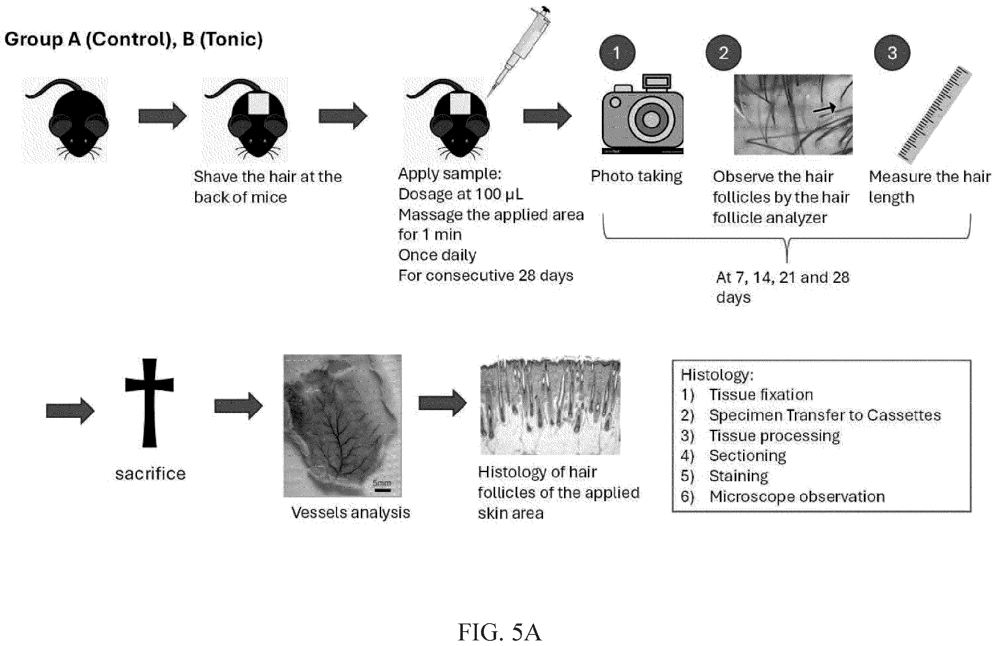

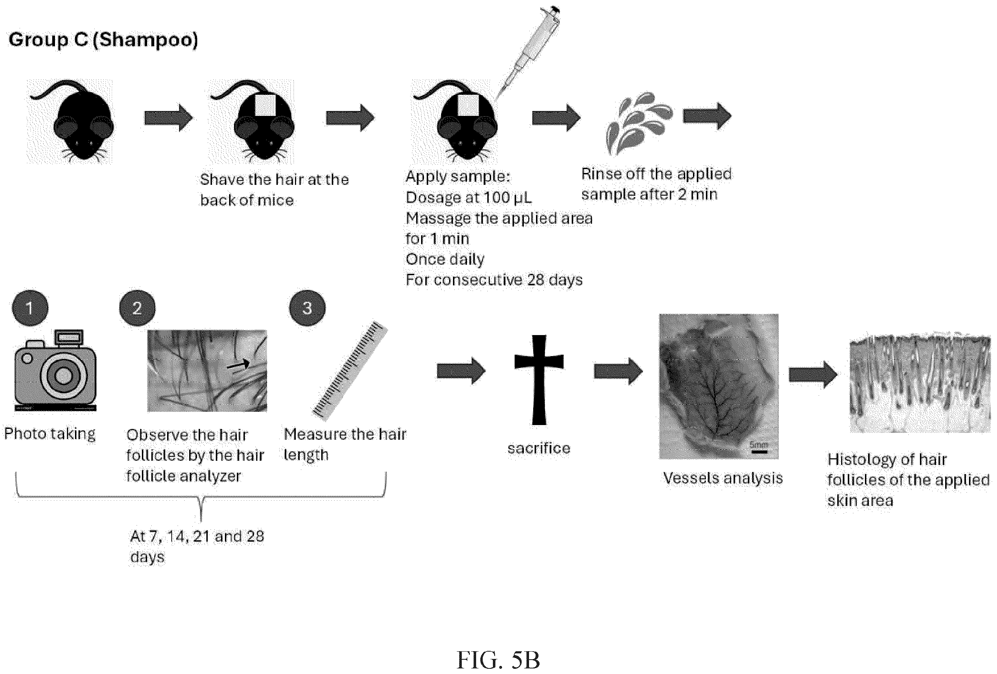

Twenty mice were randomly divided into 5 groups (Groups A to E) with 4 mice per group. Each group was treated with a different product as shown in Table 5 and FIG. 4, according to the procedures illustrated in FIGS. 5A and 5B.

**Table 5. Treatments for Mice in Groups A to E**

| **Mouse Group (n=4)** | **Treatment** |
|---|---|
| A | Water (100 µL) |
| B | Nano Hair tonic (100 µL) |
| C | Nano Hair shampoo (100 µL) |
| D | Competitor product I (100 µL) |
| E | Competitor product II (100 µL) |

### 4.4 Methods

*For mice in Groups A, B, D, E shown in Table 5, the following assessment procedures were performed:*
Now referring to FIG. 5A. The hair at the back of the mice was shaved, and a sample of the appropriate haircare product was applied topically on the shaved area at their predetermined dosage (see Table 5). The haircare product was gently massaged into the applied area for a duration of 1 minute. This process was repeated once daily for a consecutive period of 28 days. On Day 0, 7, 14, 21, and 28, photographs were taken to document the changes in hair and follicles. The follicles were analyzed using a hair follicle analyzer. Hair length was measured. Mice were then sacrificed for vessel analysis and hair follicle histology of the treated skin area.

*For mice in Group C shown in Table 5, the following assessment procedures were performed:*
Now referring to FIG. 5B. The hair at the back of the mice was shaved, and 100 µL Nano Hair shampoo was applied to the shaved area (see Table 5). The shampoo was gently massaged into the applied area for a duration of 1 minute. The shampoo was then rinsed off with water for 2 minutes after massage. This process was repeated once daily for a consecutive period of 28 days. On Day 0, 7, 14, 21, and 28, photographs were taken to document the changes in hair and follicles. The follicles were analyzed using a hair follicle analyzer. Hair length was measured. Mice were then sacrificed for vessel analysis and hair follicle histology of the treated skin area.

*For all groups (Groups A-E), the hair growth was evaluated by the following procedures:*
On Day 0, 7, 14, 21, and 28, pictures of the back of each mouse were taken using a digital camera (iPhone 13). To facilitate evaluation of the effect on hair growth, the hair length was measured and the hair follicles of each mouse were observed. Images of the back of each mouse were digitalized using ImageJ (National Institutes of Health and the Laboratory for Optical and Computational Instrumentation, USA) to obtain the corresponding grayscale values for the semi-quantified evaluation of the hair growth effects in the applied region.

To observe hair growth, a piece of skin on the back (about 2x2 cm²) was excised using sterilized surgical scissors after sacrificing the mice. Skin specimens were immersed in Sucrose-PBS solution (30%) for 24 hours and stored in 4% Paraformaldehyde Fix Solution at room temperature for 24 h for fixation followed by mounting in OCT embedding compound and subsequent storage at -80°C. The embedded tissue was sectioned into 10 µm sections, stained with Haematoxylin and Eosin, and observed under an optical microscope at 10x magnification.

### 4.5 Results

### 4.5.1 Hair growth observed in groups after treatment with Nano Tonic or Shampoo

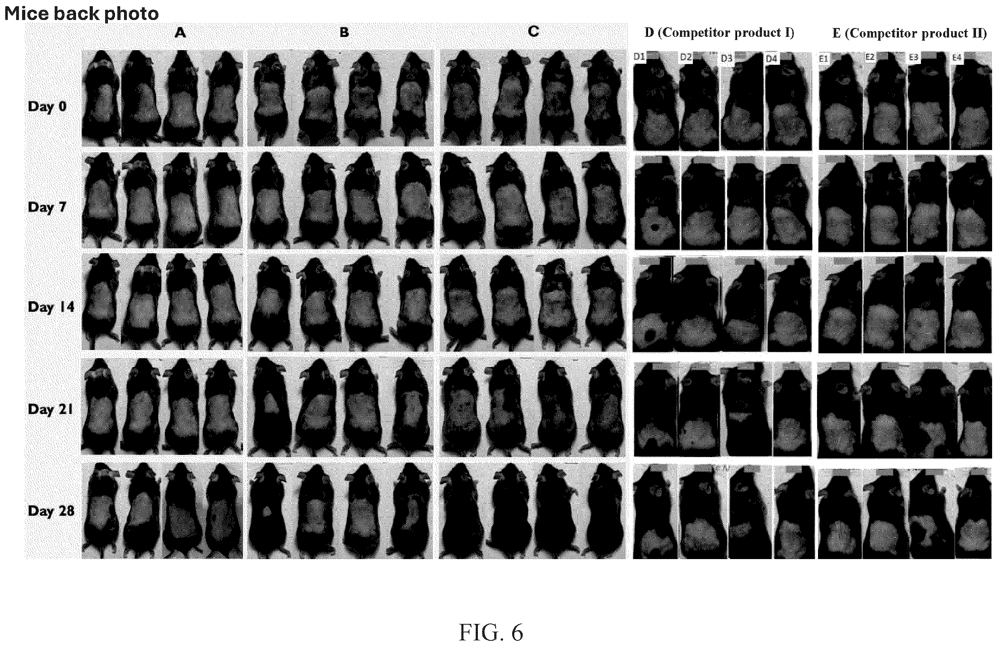

On Day 0, 7, 14, 21, 28, photos of each mouse were taken before applying the products. The results of the five studied groups (Groups A-E) are shown in FIG. 6. To quantitatively present the hair growth effects, the mean grayscale values in the applied region were calculated using ImageJ and were used as an indicator for the hair growth effects with a lower grayscale value representing more profuse hair growth. It is noted that the grayscale values in Groups B and C significantly decreased with time, while those in Groups A, D and E remained the same during the period of measurement, suggesting the hair growth effects of Groups B and C outperformed the rest of the products.

### 4.5.2 Mice in groups treated with Nano Tonic or Shampoo demonstrated increased hair lengths

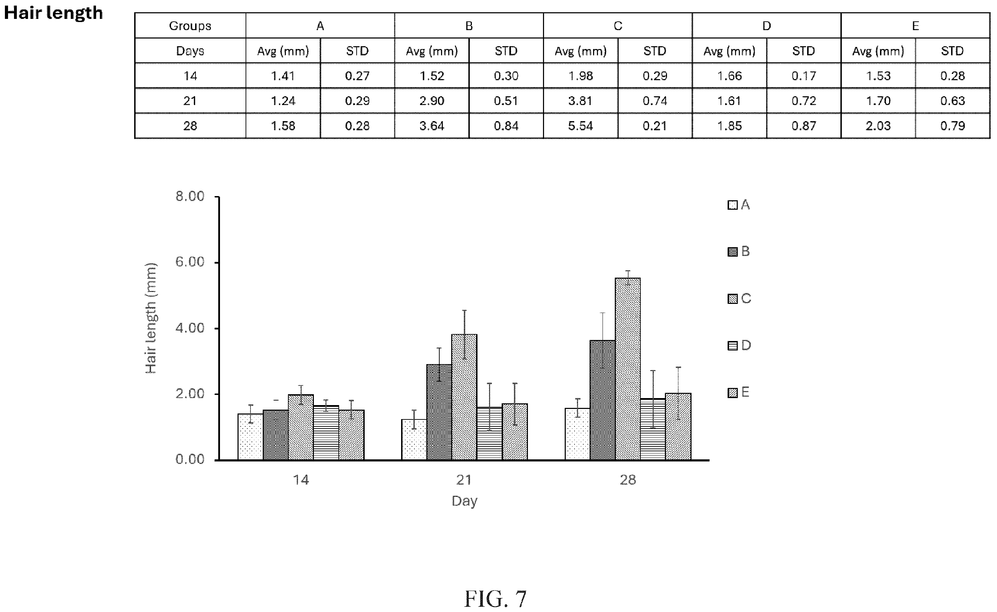

Since the hair length of the studied mice was too short to be measured in the first week, only the hair length values measured on Days 14, 21 and 28 are shown. Records of changes in hair length on Days 14, 21, and 28 are illustrated in Table 6 and FIG. 7.

**Table 6. Hair length on Days 14, 21, and 28**

| | Mouse Mean Hair Length (mm), N=6 | | | | | |
|---|---|---|---|---|---|---|
| | Day 14 | | Day 21 | | Day 28 | |
| | Mean | SD | Mean | SD | Mean | SD |
| Control (Water; Group A) | 1.41 | 0.27 | 1.24 | 0.29 | 1.58 | 0.28 |
| Nano Hair Tonic (Group B) | 1.52 | 0.30 | 2.90 | 0.51 | 3.64 | 0.84 |
| Nano Hair Shampoo (Group C) | 1.98 | 0.29 | 3.81 | 0.74 | 5.54 | 0.21 |
| Competitor product I (Group D) | 1.66 | 0.17 | 1.61 | 0.72 | 1.85 | 0.87 |
| Competitor product II (Group E) | 1.53 | 0.28 | 1.70 | 0.63 | 2.03 | 0.79 |

The results as depicted in Table 6 and Figure 7 demonstrated significant improvements in hair growth over the 28-day period for both Nano Hair Shampoo (Group C) and Nano Hair Tonic (Group B). Nano Hair Shampoo (Group C) showed a remarkable percentage increase of 179.80% in hair length from Day 14 to Day 28. Nano Hair Tonic (Group B) exhibited a percentage increase of about 139.47% over the same duration. The water control (Group A) only showed a percentage increase of 12.06% in hair length.

In comparison, the competitor products and the control group showed relatively lower efficacies. Competitor Product II (Group E) demonstrated an increase of around 32.68%, while Competitor Product I (Group D) showed an increase of approximately 11.45%. In addition, intergroup comparisons in mice hair lengths indicated that 21- and 28-day treatment of Nano Hair Tonic (Group B) and Nano Hair Shampoo (Group C) all showed significant increases compared with those from the control group (Group A) and Competitors' products I and II (Groups D and E).

### 4.5.3 Hair follicle analysis for groups using Nano Tonic or Shampoo

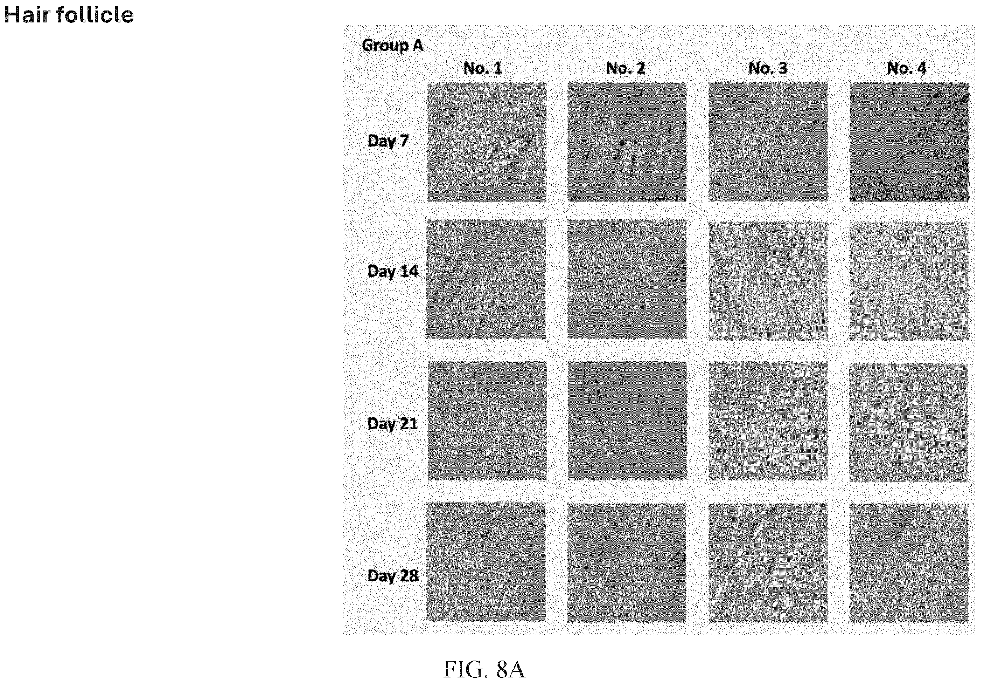

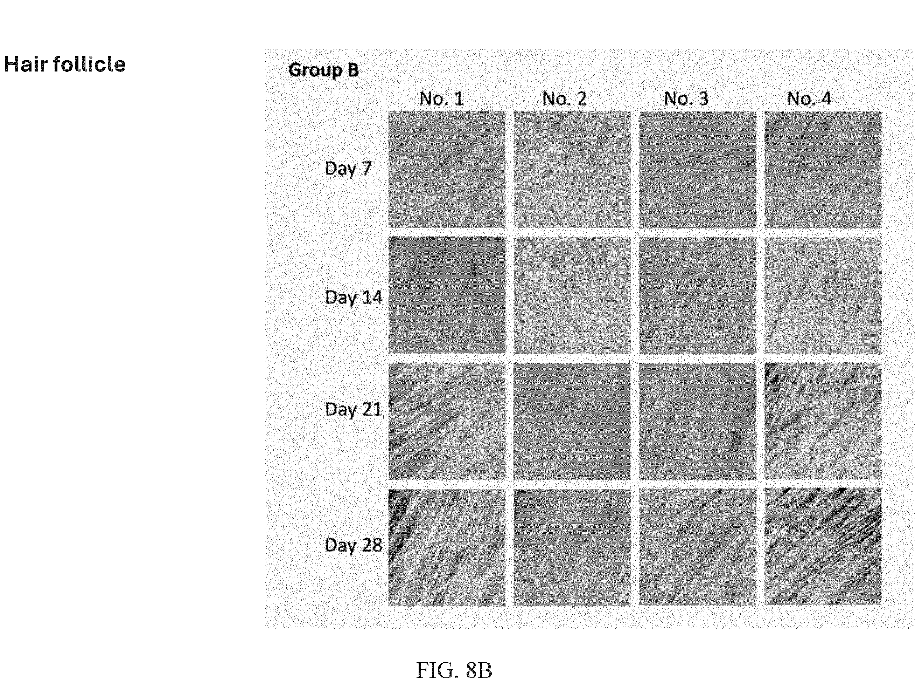

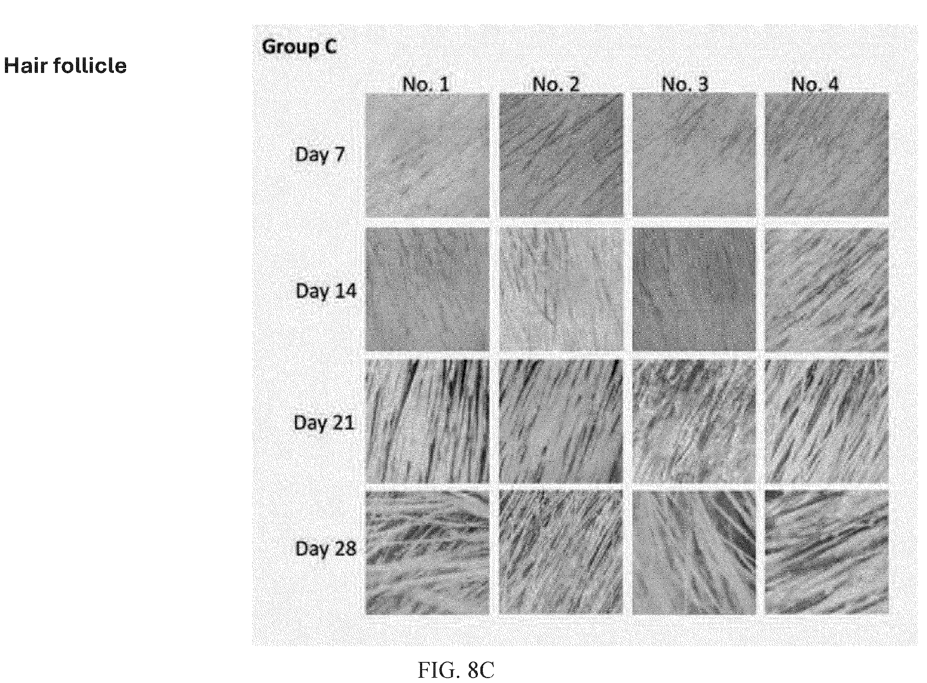

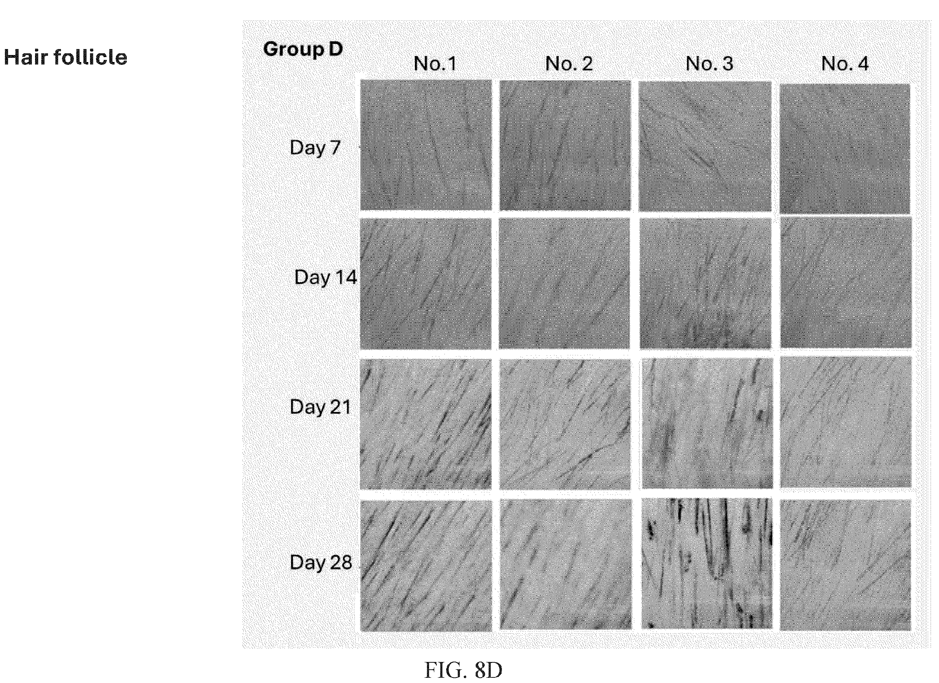

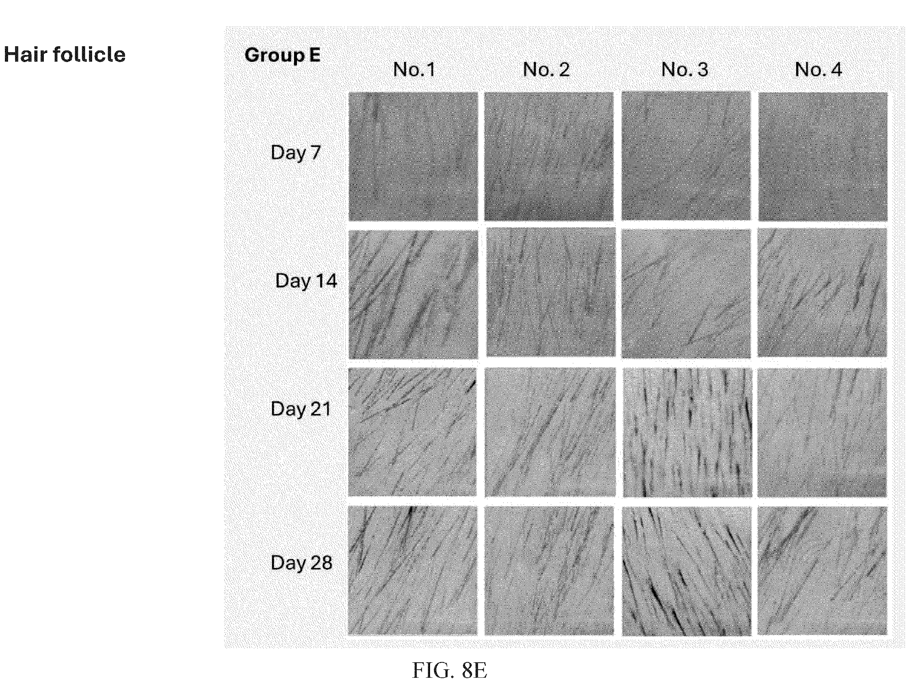

Hair follicle analysis of mice in all five groups (Groups A-E) on Days 7, 14, 21 and 28 were performed. The results are shown in FIGS 8A-E. Compared with the hair follicles of the control group (FIG. 8A), only those of the groups treated with the Nano Hair Tonic (Group B) and the Nano Hair Shampoo (Group C) on Days 21 and 28 showed a trend of increase.

### 4.5.4 Histological analysis for groups treated with Nano Tonic or Shampoo

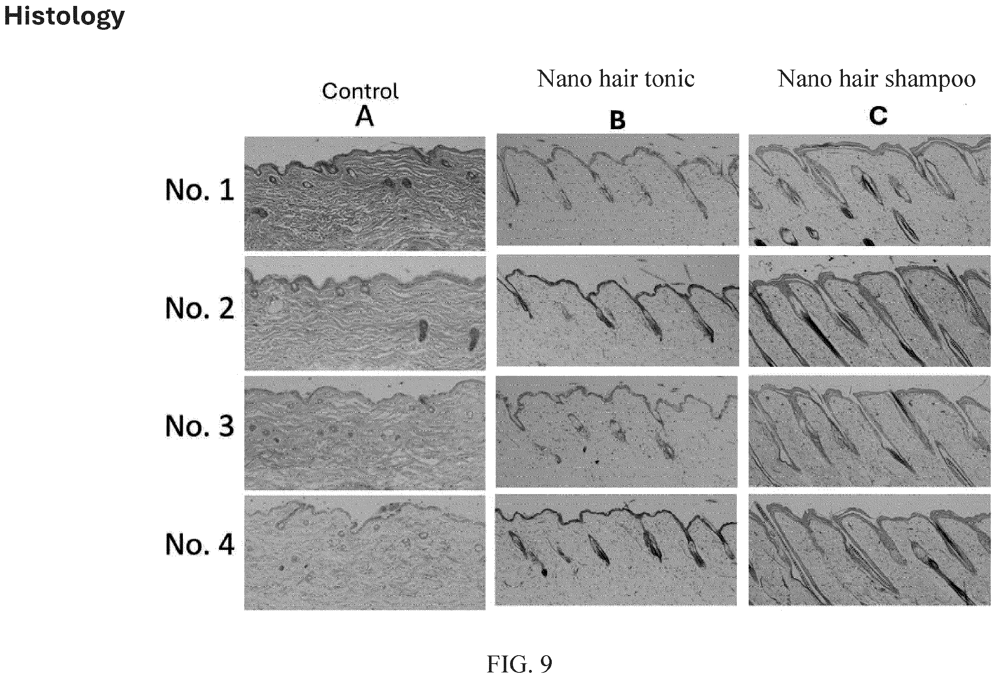

Hair follicle morphology of mice from three treatment Groups A-C on Day 28 was analyzed based on representative hair follicle histology images thereof. The results are shown in FIG 9. The Nano Hair Tonic (Group B) showed a higher density of hair follicles than that of the control group (Group A). The Nano Hair Shampoo (Group C) demonstrated the highest density of hair follicles among the three treatment groups. These findings support the efficacy of both Nano Hair Shampoo (Group C) and Nano Hair Tonic (Group B) in enhancing hair growth.

### 4.6 Conclusion

The above results indicate that both Nano Hair Tonic and Hair Shampoo (Groups B and C) could promote hair growth and outperform those of the control group (Group A), Competitor product I (Group D) and Competitor product II (Group E) in the mouse model.

### Example 5: Methods of use of Nano Tonic and Nano Shampoo

To maximize the effect of Nano Tonic to users, the users are advised to apply 3-5 ml of the tonic over the scalp, followed by gentle and thorough massage for at least 1 minute. There is no need to rinse off. It is recommended to use the Tonic twice a day.

To maximize the effect of Nano Shampoo to users, the users are advised to rub an appropriate amount of shampoo gently onto wet hair and massage the scalp for at least 1 minute, followed by rinsing with water. To address hair loss concerns, it is recommended to use the products once every day. For overall hair health maintenance, a frequency of application of 2-3 times per week is suggested.

For combo use of both Nano Shampoo and Nano Tonic, users are advised to apply an appropriate amount of shampoo dosage on hair scalp and massage the scalp for at least 1 minute, followed by rinsing with water. Then the users are advised to further apply an appropriate amount of tonic dosage on the scalp and massage the scalp for at least 1 minute. No need to rinse off the tonic.

The exemplary embodiments of the present invention are thus fully described. Although the descriptions refer to particular embodiments, it will be clear to one skilled in the art that the present invention may be practiced with variation of these specific details. Hence this invention should not be construed as limited to the embodiments set forth herein.

## Claims

1. A hair tonic composition, comprising all or part of active ingredients below:
a plurality of nanosized particles, the nanosized particles comprises:
*Phyllanthus emblica* Fruit Extract;
*Zingiber officinale* (Ginger) Root Extract;
*Sophora flavescens* Extract;
*Eclipta prostrata* Extract;
*Lithospermum erythrorhizon* Root Extract;
*Bacopa monnieri* Extract;
*Carthamus tinctorius* (Safflower) Flower Extract;
*Polygoni multiflori radix praeparata* Extract;
*Ligustrum lucidum* Extract;
*Panax ginseng* Root Extract;
*Mentha arvensis* Leaf Oil;
Monosodium Glutamate;
Cyanocobalamin;
Caffeine;
Rosemary Oil; and
Menthol.

2. The hair tonic composition of claim 1, further comprising Arginine.

3. The hair tonic composition of claims 1 or 2, wherein the active ingredients further comprise one or more ingredients selected from a group consistent of:
Olive Oil;
Grapeseed Oil;
Ginger Oil;
Chamomile Oil;
Greentea Oil;
Cedar Oil;
Brown Seaweed Extract;
*Dendrobii officinalis caulis* Extract;
*Poria cocos* Extract;
*Eucommia ulmoides* Bark Extract;
Fucoidan;
Fucoanxanthin;
Amla; and
Zinc Glycinate.

4. A hair tonic composition, comprising nano vesicles from all or part of below:
Unsaturated Phosphoslipids;
Hydrogenated Phospholipids;
Lecithins;
Phosphatidylserine;
Phosphatidylcholine;
Phosphatidylglycerols;
Phosphatidylethanolamines;
Phosphatidic Acids;
Pegylated Phospholipids; and
Glycerophosphocholine.

5. A hair tonic composition, comprising penetration enhancers from all or part of below:
Glycerol;
Ethyl Alcohol;
Propyl Alcohol;
Propylene Glycol;
Butylene Glycol;
Pentylene Glycol;
Hexylene Glycol;
Polysorbate 20;
Polysorbate 60;
Polysorbate 80; and
Polyethylene Glycol 300.

6. A hair tonic composition, comprising pH modulators from one or more of below: Ethylene Diamine; Arginine; Citric Acid; Ascorbic Acid; Sodium Ascorbate; and Sodium Hydroxide.

7. The hair tonic composition of any one of claims 1-6, wherein the hair tonic composition is an intermediate hair tonic composition or final hair tonic composition.

8. The hair tonic composition of any one of claims 1-7, wherein the plurality of nanoparticles is 10 - 1.0 × 10⁵ nm in diameter.

9. A method of producing a hair tonic composition, comprising the steps of:
a) nanosizing a first set of components with a high-pressure homogenizer to obtain a plurality of nanoparticles,
wherein the components comprise the hair tonic composition of any one of claims 1, 3-5;
and
b) adding the first set of components to a second set of components to produce the intermediate hair tonic composition or final hair tonic composition;
wherein the nanosizing is performed with the high-pressure homogenizer at 100-1000 bar for 1-10 cycles.

10. The method of claim 9, wherein the hair tonic composition further comprises Arginine.

11. The method of claims 9 or 10, wherein the hair tonic composition further comprises the hair tonic composition of claim 6.

12. The method of any one of claims 9-11, wherein the first set of components is filtered via a 40-230 mesh test sieve before nanosizing.

13. The method of producing the hair tonic composition of any one of claims 9-12, prior to step a), the method comprises the steps of:
i) adding aqua to a first mixing tank and then adding individually a first subset of the first set of components under stirring at 50-400 rpm at 10-50 °C, producing a first mixture;
ii) adding a second subset of the first set of components to the first mixing tank with gentle mixing at 50-400 rpm, and then warming the first mixing tank to 30-80 °C and homogenizing at 2000-15,000 rpm, producing a second mixture;
iii) adding individually a third subset of the first set of components to a second mixing tank and heating up to 30-80 °C and mixing at 50-400 rpm, producing a third mixture;
iv) pouring the third mixture to the second mixture and then stirring at 50-400 rpm at 30-80 °C for 30 minutes, producing a fourth mixture;
v) filtering the fourth mixture via a 40-230 mesh test sieve; and
wherein the nanosizing is performed at 100-1000 bar for 1-10 cycles.

14. The method of producing the hair tonic composition of any one of claims 9-13, wherein in step b), the method comprises the steps of:
vi) adding a first subset of the second set of components to the fourth mixture homogenized with the high-pressure homogenizer with gentle mixing at 50-400 rpm, producing a fifth mixture; and
vii) adding a second subset of the second set of components to the fifth mixture to adjust the pH to 4-8, and discharging to produce the hair tonic composition.

15. The method of any one of claims 9-14, wherein the plurality of nanoparticles is 10 - 1.0 × 10⁵ nm in diameter.

16. The method of claim 15, wherein the plurality of nanoparticles is 122-1000 nm in diameter.

17. A hair tonic composition for improving hair growth or preventing hair loss, wherein the intermediate hair tonic composition or final hair tonic composition comprises:
0.01-1.00% Monosodium Glutamate;
0.01-1.00% Chitosan;
1.00-10.00% *Phyllanthus emblica* Fruit Extract;
1.00-10.00% *Zingiber officinale* Root Extract;
1.00-10.00% *Sophora flavescens* Extract;
1.00-10.00% *Eclipta prostrata* Extract;
0.01-10.00% *Lithospermum erythrorhizon* Root Extract;
0.01-10.00% Cyanocobalamin;
0.01-10.00% Caffeine;
0.01-10.00% *Bacopa monnieri* extract;
0.01-10.00% *Carthamus tinctorius* flower extract;
0.01-10.00% *Polygoni multiflori radix praeparata* extract;
0.01-10.00% *Ligustrum lucidum* extract;
0.01-10.00% *Panax ginseng* root extract;
0.05-10.00% Lecithin;
0.01-10.00% *Mentha arvensis* Leaf Oil;
0.01-10.00% PEG-40 Hydrogenated Castor Oil;
1.00-5.00% Polysorbate 20;
0.01-10.00% Rosemary Oil;
0.01-10.00% Menthol;
1.00-10.00% Glycerin;
1.00-10.00% Butylene Glycol;
0.10-10.00%
Ethylhexylglycerin;
Capryloyl Glycine;
1,2-Hexanediol; and
Butylene Glycol;
0.10-10.00% Pentylene Glycol; and
0.01-10.00% Arginine;
and water adding up to 100%.

18. The hair tonic composition of claim 17, wherein the hair tonic composition is produced by a process comprising the steps of:
a) nanosizing a first set of components with a high-pressure homogenizer to obtain a plurality of nanoparticles,
wherein the components comprise:
*Phyllanthus emblica* Fruit Extract;
*Zingiber officinale* (Ginger) Root Extract;
*Sophora flavescens* Extract;
*Eclipta prostrata* Extract;
*Lithospermum erythrorhizon* Root Extract;
*Bacopa monnieri* Extract;
*Carthamus tinctorius* (Safflower) Flower Extract;
*Polygoni multiflori radix praeparata* Extract;
*Ligustrum lucidum* Extract;
*Panax ginseng* Root Extract;
*Mentha arvensis* Leaf Oil;
Monosodium Glutamate;
Cyanocobalamin;
Caffeine;
Rosemary Oil;
Menthol;
and
b) adding the first set of components to a second set of components to produce the intermediate hair tonic composition or final hair tonic composition;
wherein the nanosizing is performed with the high-pressure homogenizer at 100-1000 bar for 1-10 cycles.

19. Use of an effective amount of the hair tonic composition of any one of claims 1-8, 17-18 or the hair tonic composition produced by the method of any one of claims 9-16 on hair scalp, wherein after the hair tonic composition is applied on the hair scalp, the hair scalp is massaged for at least 1 minute.

20. The use of claim 18, further comprising an effective amount of a hair shampoo composition on the hair scalp.

21. Use of an effective amount of a combination of the hair tonic composition of any one of claims 1-8, 17-18 or the hair tonic composition produced by the method of any one of claims 9-16 and a hair shampoo composition on hair scalp, wherein after the hair shampoo composition is applied on the hair scalp, the hair scalp is massaged for at least 1 minute, followed by rinsing the hair scalp with water; then the hair tonic composition is applied on the hair scalp, the hair scalp is massaged for at least 1 minute.
